# EUROPEAN PATENT SPECIFICATION

(11) **EP 4 508 026 B1**
(45) Date of publication and mention of the grant of the patent: **19.11.2025**
(21) Application number: 23717981.7
(22) Date of filing: 13.04.2023
(51) Int. Cl.: C07C 273/00, F28D 3/02, F28D 3/04

(54) **A FALLING FILM HEAT EXCHANGER**
FALLFILMWÄRMETAUSCHER
ÉCHANGEUR DE CHALEUR À RUISSELLEMENT

(30) Priority: 13.04.2022 EP 22168206
(43) Date of publication of application: 19.02.2025
(73) Proprietor: Yara International ASA, 0277 Oslo (NO)
(72) Inventor: PORRO, Lino Giovanni, 1040 Etterbeek (BE)
(74) Representative: De Clercq & Partners
(86) International application number: PCT/EP2023/059609
(87) International publication number: WO 2023/198802

(56) References cited:
- EP-A1- 1 469 269
- AU-A1- 2020 311 257
- LT-B- 5 482
- US-A1- 2012 097 378
- US-A1- 2022 064 109

## Description

### Field of the invention

The present disclosure is related to chemical engineering, in particular to the field of heat exchangers. AU 2020 311 257 A1 discloses a heat exchanging section for a vertical falling film heat exchanger comprising:- a cylindrical body, comprising a top compartment and a bottom compartment separated from each other by a first disk comprising a plurality of openings, wherein the top compartment has an open top end and the first disk at its bottom end, and wherein the bottom compartment has the first disk as its top end and a bottom plate comprising a plurality of openings as its bottom end, wherein the top compartment of the cylindrical body is configured to hold a liquid composition, and the bottom compartment is configured to receive a heating or cooling fluid, wherein the bottom compartment comprises a fluid inlet for a heating or cooling fluid, and a fluid outlet for a heating or cooling fluid;- a plurality of vertical tubes located within the cylindrical body, the tubes being cylindrical with a constant diameter and parallel with each other, wherein each vertical tube is received through an opening of the first disk and through an opening of the bottom plate and wherein each opening of the first disk and each opening of the bottom plate contains a vertical tube, so that the top compartment and the bottom compartment are hermetically separated from each other; and- a plurality of liquid dividers, wherein a liquid divider is located on top of each vertical tube of the plurality of vertical tubes wherein- each liquid divider has an open top end;- each liquid divider comprises a cylindrical body, wherein the cylindrical body of each liquid divider comprises one or more openings. This disclosure only shows one heat exchanging section in the sense of the invention.

### Background of the invention

In chemical plants, a heat exchanger is a device that allows to heat up or cool down a fluid, i.e. a liquid or a gas, without direct contact between the fluid and the cooling or heating medium.

There are different designs of such devices, and one of them is the falling film shell-and-tube heat exchanger, hereafter named falling film heat exchanger.

A shell-and-tube heat exchanger is a device comprising three sections: a distributing section, wherein the fluid to be cooled or heated is introduced into the device and separated in sub-streams, a heat exchanging section, which comprises a body and a plurality of tubes, wherein the plurality of tubes directs the fluid from the distributing section to a collecting section wherein the cooled or heated fluid is recovered. The heat exchanging section comprises two spaces: the shell space is the continuous space between the body and the plurality of vertical tubes, wherein the heating or cooling medium is circulated, and the tube space, which is the discontinuous space formed by the collection of the inside of each tube. Typically, the tubes are evenly distributed across the cross section of the shell in a shell-and-tube heat exchanger.

A falling film exchanger is a particular type of shell-and-tube heat exchanger, wherein the exchanger is a vertical top-to-bottom exchanger. The liquid to be heated up or cooled down is directed to the distributing section, which sits at the top of the heat exchanging section. The distributing section comprises a plurality of ferrules or liquid dividers, wherein a liquid divider sits on the top end of each tube of the heat exchanging section. The liquid dividers are designed such that the liquid in the distribution section is directed in the plurality of tubes and creates a film coating the internal circumference of the tube, and falls towards the collection section by gravity. The liquid enters the heat exchanging section in the tubes and exchanges heat with the sides of the tubes that are heated up or cooled by the heating or cooling medium circulating in the shell space of the heat exchanging section. If the heat exchanger is designed to heat up the fluid, some volatile compounds comprised in the liquid may evaporate from the liquid. The gases can flow upwards in the central section of the tubes, and reach the distributing section, wherein a gas outlet allows the gases to exit the exchanger.

A falling film exchanger can be used with different liquids in different industries. One possible application in the fertilizer industry, in particular in the field of urea production, is a carbamate decomposer. A urea-producing plant comprises one or more streams of an aqueous solution comprising ammonium carbamate (NH₄(H₂NCO₂)), urea, and optionally free ammonia. Ammonium carbamate is an intermediate product in the synthesis of urea, and needs to be removed from ureacontaining streams, to obtain a pure urea solution. When heated up, ammonium carbamate decomposes into ammonia and carbon dioxide, which are gases at temperatures and pressures used in urea-producing plants. A falling film exchanger can thus be used to remove all of or part of the ammonium carbamate comprised in an aqueous solution comprising urea. The falling film exchanger uses a heating medium, such as steam or saturated steam, in its heat exchanging section to heat up the aqueous solution comprising urea and ammonium carbamate inside the plurality of tubes. The ammonium carbamate breaks down into ammonia and carbon dioxide, which escape the tube via its central section and leave the heat exchanger via a gas outlet. The ammonia and carbon dioxide can be later directed to the synthesis section of the plant to produce more urea.

It may be desirable to increase the exchange surface of the heat exchanging section of a falling film exchanger, which can be done by increasing the number of tubes or increasing the length of the tubes. This increase in surface may allow the exchanger to remove more carbamate from an aqueous solution, or process a larger flow of aqueous solution, or allow the exchanger to operate with a lower steam pressure and/or temperature.

EP1469269A1 (Casale, 2004), DE899795C (Hoechst Ag, 1953) and US3016067 (Henry Vogt Machine Company, 1962) disclose a falling film exchanger, wherein a second heat exchanging section can be connected to the existing heat exchanging section to increase the length of the heat exchanger. The three documents disclose that the second heat exchanging section comprises liquid dividers with a closed top.

### Summary of the invention

It was observed that the design of the prior art devices, such as EP1469269A1, had a major design issue: the flow of gases escaping the tubes of the bottom heat exchanging section crosses with the liquid flow falling from the top heat exchanging section, which causes disturbance in the flow of the liquid and of the escaping gas. This can create an irregular distribution of the gas flow in the vertical tubes of the top heat exchanging section, which in turn decreases the performance of the device.

The present inventor has designed a new type of heat exchanging section for extending a falling film exchanger, such that the flows of liquid and gas inside a tube do not cross each other.

According to the invention, there is provided a falling film heat exchanger comprising a distribution section, a first heat exchanging section comprising a first set of vertical tubes, a second heat exchanging section, and a collection section, wherein the first and second heat exchanging section comprises:
- a cylindrical body, comprising a top compartment and a bottom compartment separated from each other by a first disk comprising a plurality of openings, wherein the top compartment has an open top end and the first disk at its bottom end, and wherein the bottom compartment has the first disk as its top end and a bottom plate comprising a plurality of openings as its bottom end, wherein the top compartment of the cylindrical body is configured to hold a liquid composition, and the bottom compartment is configured to receive a heating or cooling fluid, wherein the bottom compartment comprises a fluid inlet for a heating or cooling fluid, and a fluid outlet for a heating or cooling fluid;
- a plurality of vertical tubes located within the cylindrical body, the tubes being cylindrical with a constant diameter and parallel with each other, wherein each vertical tube is received through an opening of the first disk and through an opening of the bottom plate and wherein each opening of the first disk and each opening of the bottom plate contains a vertical tube, so that the top compartment and the bottom compartment are hermetically separated from each other;
wherein:
- the second heat exchanging section comprises a plurality of liquid dividers, wherein a liquid divider is located on top of each vertical tube of the plurality of vertical tubes of the second heat exchanging section;
- each liquid divider of the second heat exchanging section has an open top end;
- each liquid divider of the second heat exchanging section comprises a cylindrical body, wherein the cylindrical body of each liquid divider comprises one or more openings;
- the first heat exchanging section and the second heat exchanging section comprises the same number of vertical tubes; and
- each vertical tube of the second heat exchanging section is located directly below a vertical tube of the first heat exchanging section.

Also according to the invention, there is provided a method for heating up or cooling down a liquid composition, comprising the steps of: a) providing a falling film heat exchanger according to the present disclosure; b) providing a heating fluid or a cooling fluid to the inlets of the heat exchanging sections of the falling film heat exchanger; c) providing the liquid composition to the inlet of the distribution section of the falling film heat exchanger, thereby heating or cooling the liquid composition in the heat exchanging section of the falling film heat exchanger; d) collecting the heated or cooled liquid composition from the collection section of the falling film heat exchanger.

In another aspect of the invention there is provided a method for removing ammonium carbamate from an aqueous composition, comprising the steps of: a) providing a falling film heat exchanger according to the present disclosure; b) providing a heating fluid to the inlets of the heat exchanging sections of the falling film heat exchanger; c) providing the aqueous composition comprising ammonium carbamate to the inlet of the distribution section of the falling film heat exchanger, thereby heating the aqueous composition comprising ammonium carbamate in the heat exchanging section of the falling film heat exchanger; d) collecting an aqueous composition depleted of ammonium carbamate from the collection section of the falling film heat exchanger.

### Brief description of the figures

The following description of the figures of a specific embodiment of a system according to the present disclosure is only given by way of example and is not intended to limit the present explanation, its application or use. In the figure, identical reference numerals refer to the same or similar parts and features.
Figure 1 represents a conventional falling film heat exchanger according to the prior art.
Figure 2 represents an embodiment of a heat exchanging section according to the present disclosure.
Figure 3 is detailed representation of the top of a tube of a heat exchanging section according to the present disclosure.
Figure 4 represents an embodiment of a liquid divider that can be used in a heat exchanging section according to the present disclosure.
Figure 5 shows a liquid divider according to figure 4 on top of a tube in a heat exchanging section according to the present disclosure.
Figure 6 represents an embodiment of a falling film heat exchanger according to the present disclosure.
Figure 7 is an enlarged view of the junction between the two heat exchanging sections of a falling film heat exchanger according to the present disclosure.
Figure 8 is an embodiment of a second disk that may be comprised in a heat exchanging section according to the present disclosure.
Figure 9 shows another embodiment of a liquid divider according to the present disclosure on top of a tube in a heat exchanging section according to the present disclosure.

### Detailed description of the invention

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

All references cited in this description are hereby deemed to be incorporated in their entirety by way of reference.

As used herein, the following terms have the following meanings:
"A", "an", and "the" as used herein refers to both singular and plural referents unless the context clearly dictates otherwise. By way of example, "a section" refers to one or more than one section.

"About" as used herein referring to a measurable value such as a parameter, an amount, a temporal duration, and the like, is meant to encompass variations of +/-20 % or less, in particular +/-10 % or less, more in particular +/-5 % or less, even more in particular +/-1 % or less, and still more in particular +/-0.1 % or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "weight percent", "%wt" or "weight%", here and throughout the description unless otherwise defined, refers to the relative weight of the respective component based on the overall weight of the formulation.

In a first aspect, the present disclosure provides a heat exchanging section for a vertical falling film heat exchanger comprising:
- a cylindrical body, comprising a top compartment and a bottom compartment separated from each other by a first disk comprising a plurality of openings, wherein the top compartment has an open top end and the first disk at its bottom end, and wherein the bottom compartment has the first disk as its top end and a bottom plate comprising a plurality of openings as its bottom end, wherein the top compartment of the cylindrical body is configured to hold a liquid composition, and the bottom compartment is configured to receive a heating or cooling fluid, wherein the bottom compartment comprises a fluid inlet for a heating or cooling fluid, and a fluid outlet for a heating or cooling fluid;
- a plurality of vertical tubes located within the cylindrical body, the tubes being cylindrical with a constant diameter and parallel with each other, wherein each vertical tube is received through an opening of the first disk and through an opening of the bottom plate and wherein each opening of the first disk and each opening of the bottom plate contains a vertical tube, so that the top compartment and the bottom compartment are hermetically separated from each other; and
- a plurality of liquid dividers, wherein a liquid divider is located on top of each vertical tube of the plurality of vertical tubes; wherein:
- each liquid divider has an open top end;
- each liquid divider comprises a cylindrical body, and
- the cylindrical body of each liquid divider comprises one or more openings.

The heat exchanging section according to the present disclosure comprises many features in common with a conventional heat exchanging section of a conventional shell-and-tube heat exchanger:
- a cylindrical body comprising a fluid inlet for a heating or cooling fluid, and a fluid outlet for a heating or cooling fluid, the cylindrical body comprising an open top end and a bottom plate comprising a plurality of openings;
- a plurality of vertical tubes located within the cylindrical body, the tubes being cylindrical with a constant diameter and parallel with each other;
- the cylindrical body comprises two compartments hermetically separated from each other, a top compartment and a bottom compartment, by a first disk comprising a plurality of openings;
- the top compartment of the cylindrical body is configured to hold a liquid composition, and the bottom compartment is configured to receive a heating or cooling fluid;
- each vertical tube is received through an opening of the first disk, and through an opening of the bottom plate.
The plurality of vertical tubes is evenly distributed across a cross-section of the cylindrical body. The bottom plate and the first disk comprise a number of openings equal to the number of tubes comprised in the heat exchanging section. The first disk may be referred to as the tubesheet.

The bottom compartment comprises two spaces: the shell space is the continuous space between the body and the plurality of vertical tubes, wherein the heating or cooling fluid is circulated, and the tube space, which is the discontinuous space formed by the collection of the inside of each vertical tube.

The heat exchanging section according to the present disclosure further comprises a plurality of liquid dividers located at the top of each vertical tube. These liquid dividers ensure that, in operation, the liquid to be cooled or heated can reach inside the tubes of the heat exchanger, and form a film coating the inside of the vertical tubes; and that the gases evaporating from the liquid can freely flow to the top of a tube and through a liquid divider without crossing the path of the falling liquid. So a new type of liquid divider was designed, wherein:
- each liquid divider has an open top end;
- each liquid divider comprises a cylindrical body comprising one or more openings.
The open top end allows the gases evaporating from the liquid to flow through the liquid divider without crossing the path of the falling liquid. The one or more openings allow the liquid to flow inside the liquid divider and the tube. The cylindrical body of the liquid divider may have the same diameter or a smaller diameter than the vertical tubes.

In one embodiment, the cylindrical body of the liquid divider has a diameter equal to from 50% to 100% of the diameter than the vertical tubes.

In one embodiment, each liquid divider comprises a cylindrical body with a diameter equal or smaller than the diameter of the vertical tubes, and a top end being a truncated cone, wherein the diameter at the top of the liquid divider is less than the diameter of the cylindrical body of the liquid divider

In one embodiment, each liquid divider comprises two or more openings, such as for instance four openings, in its cylindrical body. It may be an advantage for the liquid divider to comprise more than one opening, particularly evenly spaced around the circumference of the cylindrical body in the same cross-section of the liquid divider, so that the liquid is flowing evenly into the liquid divider from several openings, and creates a film on the entire inner surface of the liquid divider in a shorter distance. In one embodiment, each liquid divider comprises at least four openings in its cylindrical body.

In one embodiment, each liquid divider comprises 1, 2, 3, 4, 5, 6, 7, 8, 9, or 10 openings.

In one embodiment, the two or more openings are comprised in the same cross-section of the liquid divider. Having the openings comprised in the same cross-section of the liquid divider ensures that the liquid always enters the liquid divider via the two or more openings at the same height.

In one embodiment, a liquid divider comprises four openings, the four openings being comprised in the same cross-section of the liquid divider and are spaced 90 °, thereby forming a cross. It was observed that a symmetrical distribution of the openings on the cross-section of the liquid divider, i.e. such that the four openings form an imaginary cross in that cross-section, allows the film to form faster inside the liquid divider.

In one embodiment, the liquid dividers comprise a second cylindrical section connected to its cylindrical body. The second cylindrical section of a liquid divider may be configured to be inserted into a vertical tube of the heat exchanging section. In that embodiment, the external diameter of the second cylindrical section is at most equal to the internal diameter of the vertical tubes of the heat exchanging section, at least 0.01 mm, at least 0.05 mm, at least 0.1 mm, at least 0.2 mm, at least 0.3 mm, at least 0.4 mm, or at least 0.5 mm smaller than the internal diameter of the vertical tubes of the heat exchanging section.

Alternatively, the second cylindrical section of the liquid divider may be configured to surround the top end of a vertical tube of the heat exchanging section. In that embodiment, the internal diameter of the second cylindrical section is at least equal to the external diameter of the vertical tubes of the heat exchanging section, at least 0.01 mm, at least 0.05 mm, at least 0.1 mm, at least 0.2 mm, at least 0.3 mm, at least 0.4 mm, or at least 0.5 mm greater than the external diameter of the vertical tubes of the heat exchanging section. This section increases the stability of the liquid divider when sitting on top of a tube.

If the second cylindrical section of the liquid divider is configured to surround the top end of a vertical tube of the heat exchanging section, the liquid divider may comprise a gasket, in particular a gasket made of a fluorinated polymer, such as polytetrafluoroethylene, also known as PTFE. The gasket is located between the tube and the second cylindrical section of the liquid divider to increase the stability of the liquid divider.

In one embodiment, the external diameter of the second cylindrical section is from 0.01 to 0.5 mm smaller to the internal diameter of the vertical tubes of the heat exchanging section.

In one embodiment, the internal diameter of the second cylindrical section is from 0.01 to 3.0 mm, from 0.01 to 1.0 mm, from 1.0 to 3.0 mm, or from 0.01 to 0.5 mm greater to the external diameter of the vertical tubes of the heat exchanging section.

In one embodiment, the heat exchanging section according to the present disclosure further comprises a second disk located above the first disk, the second disk comprising a plurality of first openings for receiving the plurality of liquid dividers, and a plurality of second openings for allowing the liquid composition to pass through the second disk. A second disk comprising two series of openings, wherein one series is configured to receive the plurality of liquid dividers and the other series is configured to let the liquid go through, increases the rigidity of the system by reducing the vibrations of the liquid dividers in operation.

In one embodiment, the series of openings of the second disk, that are configured to receive the liquid dividers, are cylindrical. The series of openings of the second disk, that are configured let a fluid go through, may have any type of design, and are typically evenly distributed across the area of the second disk.

In one embodiment, the series of openings of the second disk, that are configured let a liquid go through, are cylindrical. Openings with a cylindrical shape may be easier to manufacture.

In one embodiment, the diameter of the openings configured to receive the plurality of liquid dividers is larger than the outer diameter of the liquid dividers, such that a liquid composition may pass through the second disk. In one embodiment, the diameter of the openings configured to receive the plurality of liquid dividers is 0.05, 0.1, 0.15, 0.2, 0.3, 0.4, 0.5, 0.6, 0.7, 0.8, 0.9 or 1mm larger than the outer diameter of the liquid dividers. The larger the openings are, the more liquid composition is able to pass through.

In one embodiment, the second openings for allowing the fluid composition to pass through the second disk are cylindrical, in particular cylindrical with a diameter of from 5 to 10 mm.

In one embodiment, the inner diameter of the top end of the liquid dividers is from 10% to 80%, from 15% to 80%, from 20% to 80%, from 10% to 70%, or from 20% to 70% smaller than the inner diameter of the cylindrical body of the liquid dividers. The diameter should be smaller than the diameter of the tubes, so that the liquid film does not fall directly inside the liquid divider. The top end of the liquid divider should be large enough such that the gases evaporating from the tubes can flow freely out of the liquid divider without causing a pressure increase. In one embodiment, the inner diameter of the top end of the liquid dividers is equal to from 10% to 80%, from 15% to 80%, from 20% to 80%, from 10% to 70%, from 20% to 70%, from 30% to 50% of the inner diameter of the cylindrical body of the liquid dividers.

In one embodiment, the one or more openings of the liquid dividers are located from 30 to 100 mm above the first disk. The height of the openings of the liquid dividers determines the amount of liquid than can accumulate in the top compartment of the heat exchanging section.

In another aspect, the present disclosure provides a falling film heat exchanger comprising a heat exchanging section according to the present disclosure. It is possible to build a falling film heat exchanger comprising two heat exchanging sections, the first, or upper, heat exchanging section being directly above the second, or lower, heat exchanging section, with the second or lower heat exchanging section being a heat exchanging section according to the present disclosure. The falling film heat exchanger also comprises a distribution section above the upper heat exchanging section, and a collection chamber below the lower heat exchanging section.

The falling film heat exchanger comprises one inlet for a liquid to be heated up or cooled down in the distribution section, one outlet for a liquid to be heated up or cooled down in the collection section, and two inlets and two outlets for providing a heating or cooling medium to the shell side space of each exchanging section, each exchanging section comprising one inlet and one outlet fluidly connected to their body. The upper and lower heat exchanging section each comprise a plurality of vertical tubes. The two heat exchanging sections comprise the same number of vertical tubes, which are arranged in the same geometry or design, such that each tube of the upper heat exchanging section is directly above a tube of the lower heat exchanging section.

In one embodiment, the collection section of the falling film heat exchanger comprises an inlet for a passivation gas. It may be required to inject a passivation gas in the tubes of a heat exchanger to prevent or reduce corrosion. This is done by directing a stream of gas comprising oxygen gas into the collection chamber of the heat exchanger. The stream of gas travels within the vertical tubes is evacuated from the distribution chamber via a gas outlet. Without wishing to be bound by theory, the oxygen gas reacts with the tubes, which may be made of stainless steel, and create a layer of oxidized material which is resistant to chemical corrosion caused by the liquid composition, in particular if the liquid composition comprises ammonium carbamate. The passivation gas may further comprise any component selected from the group consisting of ammonia, carbon dioxide, water, and nitrogen gas.

In another aspect, the present disclosure provides a method for modifying a conventional falling film heat exchanger comprising a distribution section, a first heat exchanging section comprising a plurality of vertical tubes, and a collection section, comprising the steps of: a) providing a second heat exchanging section according to the present disclosure having the same number of vertical tubes being arranged in the same geometry as the plurality of vertical tubes of the first heat exchanging section; b) disconnecting the collection section from the first heat exchanging section; c) connecting the second heat exchanging section according to the present disclosure to the first heat exchanging section, such that each tube of the first heat exchanging section is directly above a liquid divider of the second heat exchanging section; and d) connecting the collection section to the second heat exchanging section .

A conventional falling film heat exchanger can be modified to have longer heat exchanging tubes, i.e. an increased heat transfer capacity, by introducing a heat exchanging section according to the present disclosure in a conventional heat exchanger.

The conventional falling film heat exchanger comprising a distribution section, a first heat exchanging section, and a collection section, needs to be partially dismantled: the collection section located below the first heat exchanging section needs to be disconnected from the first heat exchanging section. This operation exposes the bottom of the heat exchanging section and the lower end of the vertical tubes. A heat exchanging section according to the present disclosure is provided. The additional heat exchanging section needs to be fully compatible with the first heat exchanging section, i.e. it must have the same number of vertical tubes which are arranged in the same geometry as the existing heat exchanging section. The new heat exchanging section is connected to the first heat exchanging section, becoming the second, or lower, heat exchanging section, such that each tube of the first heat exchanging section is directly above a tube of the second heat exchanging section. This ensures that, in operation, the gases evaporating from the liquid composition in the lower heat exchanging section can freely travel upwards to the distribution section of the heat exchanger without crossing the falling liquid composition.

In one embodiment, the top end of the liquid dividers are comprised within the tubes of the first heat exchanging section. In one embodiment, the top end of the liquid dividers are below the bottom end of the tubes of the first heat exchanging section.

Finally, the collection section that was disconnected from the first heat exchanging section in step b) is connected to the lower heat exchanging section.

In one embodiment, the heat exchanging section provided in step a) has the same external diameter as the first heat exchanging section and/or of the collection chamber of the heat exchanger. If all the sections have the same external diameter, connecting the sections together may be easier and require less work.

In another aspect, the present disclosure provides a method for heating up or cooling down a liquid composition, comprising the steps of: a) providing a falling film heat exchanger according to the present disclosure; b) providing a heating fluid or a cooling fluid to the inlets of the heat exchanging sections of the falling film heat exchanger, particularly providing a heating fluid or a cooling fluid to the inlets of the bottom compartments of the first and second heat exchanging sections of the falling film heat exchanger; c) providing the liquid composition to the inlet of the distribution section of the falling film heat exchanger; d) collecting the heated or cooled liquid composition from the collection section of the falling film heat exchanger. It is understood that step c) also comprises passing the liquid composition through the plurality of vertical tubes of the first and second heat exchanging sections.

The falling film heat exchanger according to the present disclosure comprising two heat exchanging sections can be operated in a similar manner as a conventional falling film heat exchanger comprising only one heat exchanging section, with the difference that the shell side space of both heat exchanging sections must be supplied with a heating fluid, such as steam or steam condensate. The two heat exchanging sections may be supplied with the same heating fluid, for example steam at the same temperature and pressure, or a with two different heating fluids.

In one embodiment, both heating fluid inlets of the heat exchanging sections are provided with saturated steam, wherein the first inlet is provided with saturated steam at a first temperature and a first pressure, and the second inlet is provided with saturated steam at a second temperature and a second pressure.

In one embodiment, the falling film heat exchanger is a medium-pressure carbamate decomposer or a low-pressure carbamate decomposer.

In one embodiment, the heating fluid provided in step b) is saturated steam at a pressure of from 6 to 12 bars, or saturated steam at a pressure of from 3 to 5 bars.

In one embodiment, the two heat exchanging sections of the falling film heat exchanger are provided with the same heating fluid, in particular wherein the heating fluid is saturated steam, more in particular wherein the heating fluid is saturated steam at a pressure of from 3 to 12 bar.

The heating fluid is distributed to the shell space of the heat exchanging section and heat up the vertical tubes.

When the equipment is started after a break, it may be an advantage to wait after providing the heating fluid to the heat exchanging sections, such that the vertical tubes are heated up by the heating fluid and reach a desired temperature. If the liquid composition to be heated up is provided too early, the first portion thereof receives less heat when passing through the tubes than designed.

Once the equipment has reached a suitable internal temperature, the liquid composition to be heated up can be provided to the inlet of the distribution section of the heat exchanger. The distribution section ensures that the liquid composition is evenly distributed to the plurality of vertical tubes, and the liquid forms a film inside the tubes and falls due to gravity. While falling, the liquid exchanges heat with the walls of the tubes and heats up. Volatile compounds present in the liquid may evaporate and flow upwards to the distribution section. Chemical reactions may also happen as a consequence of the liquid heating up, such as the decomposition of ammonium carbamate into ammonia and carbon dioxide.

The heated liquid can then be collected from the collection section and be directed to the next processing step of the plant. The liquid composition collected from the collection section may or may not have the same chemical composition as the liquid composition that entered the distribution section.

The heat exchanger according to the present disclosure may be used as a concentrator, i.e. to evaporate water from an aqueous solution to increase the concentration of the chemical component or components comprised in the aqueous solution.

In another aspect, the present disclosure provides a method for removing ammonium carbamate from an aqueous solution, comprising the steps of: a) providing a falling film heat exchanger according to the present disclosure; b) providing a heating fluid to the inlets of the heat exchanging sections of the falling film heat exchanger, particularly providing a heating fluid to the inlets of the bottom compartments of the first and second heat exchanging sections of the falling film heat exchanger; c) providing the aqueous solution comprising ammonium carbamate to the inlet of the distribution section of the falling film heat exchanger; d) collecting an aqueous solution depleted of ammonium carbamate from the collection section of the falling film heat exchanger. It is understood that step c) also comprises passing the aqueous composition comprising ammonium carbamate through the plurality of vertical tubes of the first and second heat exchanging sections.

As mentioned above, a falling film heat exchanger may be used to purify and/or concentrate an aqueous solution comprising ammonium carbamate, and optionally urea. When a liquid composition is directed to a falling film heat exchanger, the ammonium carbamate comprised in the solution decomposes back to ammonia and carbon dioxide. Since ammonia and carbon dioxide are gases at the pressure and temperature of the heat exchanger, they evaporate from the solution in the vertical tubes. Water is also evaporated in the heat exchanger. So the aqueous solution recovered from the collection section is an aqueous solution depleted of ammonium carbamate, and optionally comprising urea.

The aqueous solution may still comprise some ammonium carbamate depending on the operating conditions, but the concentration of ammonium carbamate at the bottom of the heat exchanger is less than the concentration in ammonium carbamate at the top of the heat exchanger.

In one embodiment, the aqueous solution provided in step c) comprises from 20 to 30 weight% of ammonia, and 5 to 10 weight% of carbon dioxide. These amounts are typical for an aqueous solution provided to a medium-pressure carbamate decomposer in a urea-producing plant.

In one embodiment, the aqueous solution provided in step c) comprises from 5 to 10 weight% of ammonia, and 1 to 5 weight% of carbon dioxide. These amounts are typical for an aqueous solution provided to a low-pressure carbamate decomposer in a urea-producing plant.

In one embodiment, the aqueous solution provided in step c) comprises urea.

In one embodiment, the falling film heat exchanger provided in step a) is a low-pressure, or a medium-pressure, carbamate decomposer.

In a medium-pressure carbamate decomposer, the aqueous solution comprising urea and ammonium carbamate directed to the decomposer may have a temperature of from 135 to 155°C, or from 140 to 145 °C. In a medium-pressure carbamate decomposer, the aqueous solution depleted of ammonium carbamate and collected in the collection chamber of the decomposer may have a temperature of from 150 to 170 °C, or from 155 to 165 °C.

In a low-pressure carbamate decomposer, the aqueous solution comprising urea and ammonium carbamate directed to the decomposer may have a temperature of from 110 to 130°C, or from 115 to 125 °C. In a low-pressure carbamate decomposer, the aqueous solution depleted of ammonium carbamate and collected in the collection chamber of the decomposer may have a temperature of from 130 to 160°C, or from 135 to 150 °C.

In one embodiment, the aqueous solution collected in step d) comprises from 1 to 3 weight%, or from 1 to 2.5 weight% of ammonia, and from 0 to 1.0 weight%, or from 0.5 to 1.0 weight% of carbon dioxide. These amounts are typical for an aqueous solution collected from a low-pressure carbamate decomposer in a urea-producing plant.

In one embodiment, the aqueous solution collected in step d) comprises from 5 to 10 weight%, or from 5 to 8 weight% of ammonia, and from 1 to 5 weight%, or from 1 to 3 weight% of carbon dioxide. These amounts are typical for an aqueous solution collected from a medium-pressure carbamate decomposer in a urea-producing plant.

In another aspect, the present disclosure provides the use of the falling film exchanger according to the present disclosure for removing ammonium carbamate from an aqueous solution comprising ammonium carbamate, and urea.

In another aspect, the present disclosure provides the use of the falling film exchanger according to the present disclosure for evaporating water from an aqueous solution.

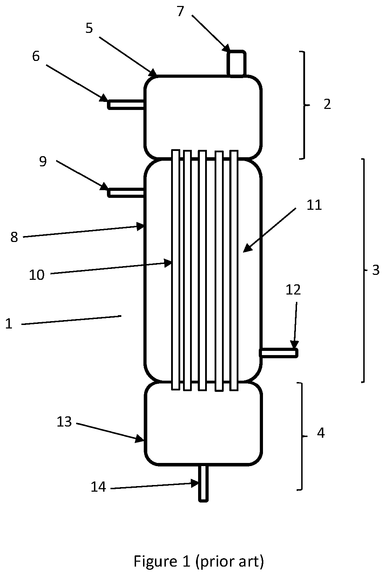

Figure 1 represents a conventional falling film heat exchanger **1.** The heat exchanger **1** comprises a distribution section **2,** a heat exchanging section **3,** and a collection section **4.** The distribution section **2** comprises a container or chamber **5,** which may have any particular geometry, such as hemi-spherical or cylindrical, an inlet **6** for a liquid composition, and an outlet **7** for gases. The heat exchanging section **3** comprises a cylindrical body **8,** an inlet **9** for a heating or cooling fluid, a plurality of vertical tubes **10,** and an outlet **12** for a heating or cooling fluid. The plurality of tubes **10** is evenly distributed across the cross section of the heat exchanging section **2.** Some tubes are omitted from the figure, such that the figure is easier to read and interpret. The tubes **10** fluidly connect the distribution section **2** and the collection section **4.** The body **8** and the tubes **10** create a continuous shell space **11,** hermetically sealed from the distribution and collection section, wherein the heating or cooling fluid can freely move. The collection section **4** comprises a body **13,** and an outlet **14** for a liquid composition.

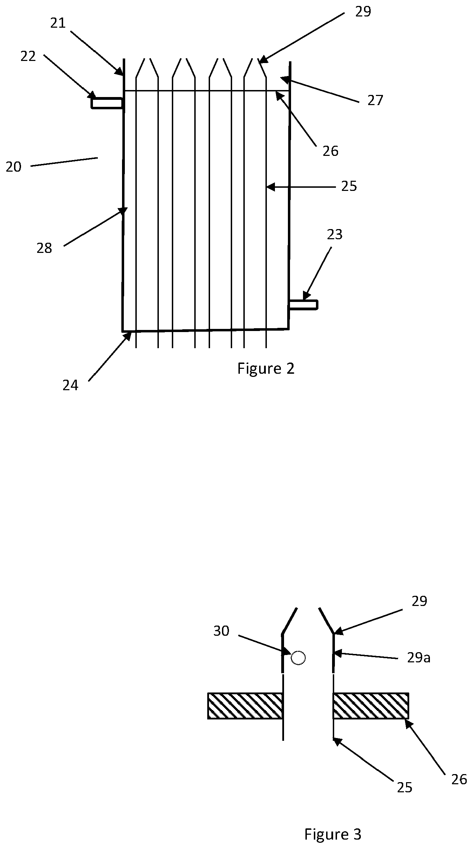

Figure 2 represents an embodiment of a heat exchanging section according to the present disclosure. The heat exchanging section **20** comprises a body **21,** in particular a cylindrical body, an inlet **22** for a cooling or heating medium, and outlet for a cooling or heating fluid **23,** a bottom plate **24** comprising a plurality of openings for receiving the plurality of tubes **25,** and a disk **26** dividing the body into two compartments hermetically separated from each other, i.e. an upper compartment **27,** and a lower compartment **28.** The heat exchanging section **20** comprises a plurality of tubes **25** distributed evenly across its cross-section. The disk **26** comprises a plurality of openings to receive the tubes **25.** The upper compartment **27** is configured to hold some of the liquid composition before it falls further in the vertical tubes **25.** Each vertical tube **25** comprise a liquid divider **29** on its top end.

Figure 3 is detailed representation of the top of a tube **25** with a liquid divider **29** of the heat exchanging section **20** shown in figure 2. The vertical tube **25** goes through the disk **26** and a liquid divider **29** is mounted on the top end of the tube **25.** The liquid divider **29** comprises a cylindrical body **29a** which has the same diameter as the tube **25,** and a top end being a truncated cone, wherein the diameter at the top of the liquid divider is smaller than the diameter of its body **29a.** The liquid divider comprises one or more openings **30** in its cylindrical body.

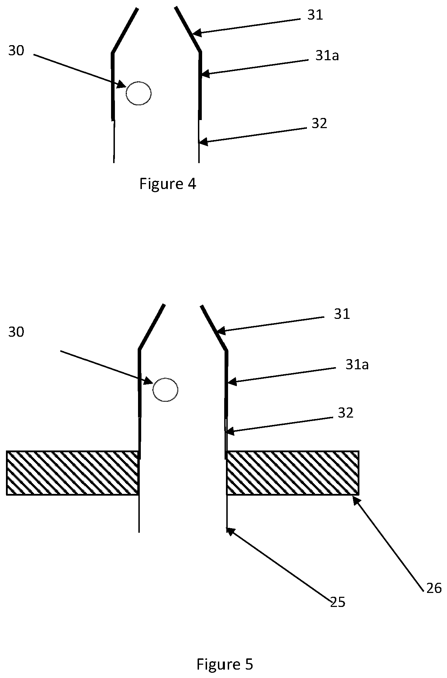

Figure 4 represents an embodiment of a liquid divider **31** that can be used in the heat exchanging section according to the present disclosure. The liquid divider **31** comprises the same features as liquid divider **29** of figure 3: a cylindrical body **31a** comprising a opening **30,** and a top end in the shape of a truncated cone. In addition, it contains a second cylindrical section **32** attached to its body **31a.** The second cylindrical section **32** has an external diameter equal to or smaller than the internal diameter of the tube **25.**

Figure 5 shows a liquid divider **31** according to figure 4 sitting on a tube **25** of a heat exchanging section. The second cylindrical section **32** sits inside the tube **25.** The section **32** increases the stability of the liquid divider **31** in the tube **25.**

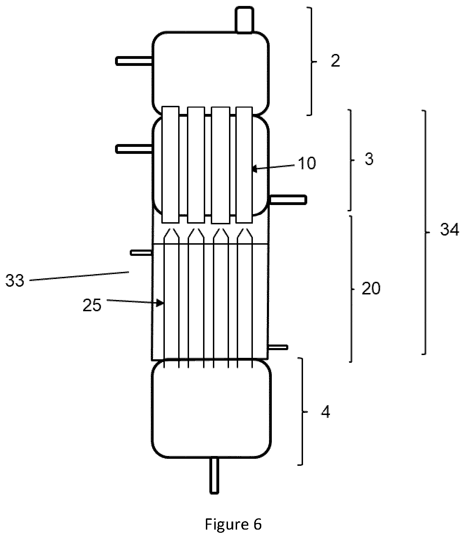

Figure 6 represents an embodiment of a falling film heat exchanger according to the present disclosure. The heat exchanger **33** comprises a distribution section **2,** a collection section **4,** and a heat exchanging module **34** comprising two heat exchanging sections, **3** and **20.** The heat exchanging section **3** is a conventional section, whereas the heat exchanging section **20** is according to the present disclosure. The tubes **10** of the section **3** are aligned with the tubes **25** of the section **20** to ensure a continuous gas flow across the entire length of the heat exchanging module **34.**

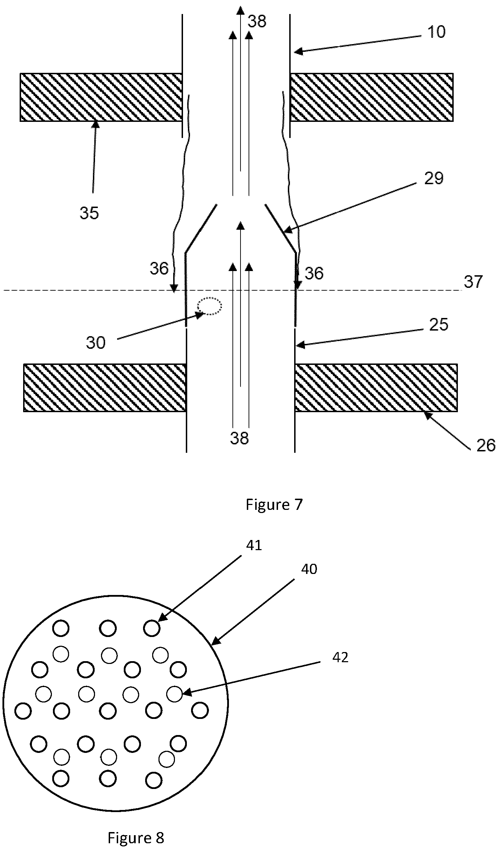

Figure 7 is an enlarged view of the area where the two exchanging sections **3** and **21** meet in the heat exchanger **33** in operation. The element **35** represents the bottom plate of the heat exchanging section **3,** and a tube **10** goes though that element **35.** The liquid composition flows downwards and is represented by the two arrows **36.** The dashed line **37** represents the liquid level in the upper compartment of the heat exchanging section in operation. The arrows **38** represent the gases evaporating from the liquid solution and rising to the top of the heat exchanger. It can be seen that the gas flow is confined to the interior of the tubes **10** and **25** at any time, while the liquid flows from the internal sides of tubes **10,** to the outside of tubes **25** and re-enter the tubes **25** via the one or more openings **30.** It can be seen that the gas and liquid flows do not cross each other.

Figure 8 is an embodiment of a second disk **40** that may be comprised in a heat exchanging section according to the present disclosure. The second disk **40** has a diameter that is at most equal to the internal diameter of the cylindrical body of the heat exchanging section. It comprises two series of openings **41** and **42.** The openings **41** are configured to receive the liquid dividers of the heat exchanging section according to the present disclosure. The openings **41** may have a diameter slightly larger, for example from 0.1 to 0.5 mm, than the diameter of the liquid dividers, such that some liquid composition may pass through the openings **41.** The openings **42** are configured to let the liquid composition flowing down the heat exchanger flow though the second disk **42.** The openings **41** and **42** are drawn with a different line thickness, so they are easier to differentiate on a drawing. However, the openings **41** and **42** may have the same or different diameters.

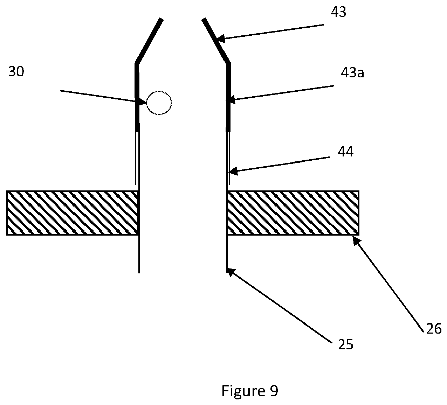

Figure 9 shows another embodiment of a liquid divider **43** according to the present disclosure sitting on a tube **25** of a heat exchanging section. The liquid divider **43** comprises a cylindrical body **43a** comprising an opening **30,** and a top end in the shape of a truncated cone. In addition, it contains a second cylindrical section **44** below its body **43a.** The second cylindrical section **44** has an internal diameter greater than the external diameter of the tubes **25.** The second cylindrical section **44** increases the stability of the liquid divider **43.** A PTFE gasket can be placed between the tube **25** and the second cylindrical section **44.**

## Claims

1. A falling film heat exchanger comprising a distribution section, a first heat exchanging section comprising a first set of vertical tubes, a second heat exchanging section, and a collection section, wherein the first and second heat exchanging section comprises:
- a cylindrical body, comprising a top compartment and a bottom compartment separated from each other by a first disk comprising a plurality of openings, wherein the top compartment has an open top end and the first disk at its bottom end, and wherein the bottom compartment has the first disk as its top end and a bottom plate comprising a plurality of openings as its bottom end, wherein the top compartment of the cylindrical body is configured to hold a liquid composition, and the bottom compartment is configured to receive a heating or cooling fluid, wherein the bottom compartment comprises a fluid inlet for a heating or cooling fluid, and a fluid outlet for a heating or cooling fluid; and
- a plurality of vertical tubes located within the cylindrical body, the tubes being cylindrical with a constant diameter and parallel with each other, wherein each vertical tube is received through an opening of the first disk and through an opening of the bottom plate and wherein each opening of the first disk and each opening of the bottom plate contains a vertical tube, so that the top compartment and the bottom compartment are hermetically separated from each other;
wherein:
- the second heat exchanging section comprises a plurality of liquid dividers, wherein a liquid divider is located on top of each vertical tube of the plurality of vertical tubes of the second heat exchanging section;
- each liquid divider of the second heat exchanging section has an open top end;
- each liquid divider of the second heat exchanging section comprises a cylindrical body, wherein the cylindrical body of each liquid divider comprises one or more openings;
- the first heat exchanging section and the second heat exchanging section comprises the same number of vertical tubes; and
- each vertical tube of the second heat exchanging section is located directly below a vertical tube of the first heat exchanging section.

2. The falling film heat exchanger according to claim 1, wherein each liquid divider of the second heat exchanging section comprises four openings in its cylindrical body, in particular wherein the four openings are comprised in the same cross-section of the liquid divider, in particular wherein the four openings are comprised in the same cross-section of the liquid divider and are spaced 90 °, thereby forming a cross.

3. The falling film heat exchanger according to claim 1 or 2, wherein the second heat exchanging section further comprises a second disk located between the open top end of the cylindrical body and the first disk of the second heat exchanging section, the second disk comprising a plurality of first openings for receiving the plurality of liquid dividers of the second heat exchanging section, and a plurality of second openings for allowing the liquid composition to pass through the second disk.

4. The falling film heat exchanger according to any one of claims 1 to 3, wherein the inner diameter of the top end of the liquid dividers of the second heat exchanging section is 20 to 80% smaller than the inner diameter of the cylindrical body of the liquid dividers of the second heat exchanging section.

5. The falling film heat exchanger according to any one of claims 1 to 4, wherein the one or more openings of the liquid dividers of the second heat exchanging section are located from 30 to 100 mm above the first disk of the second heat exchanging section.

6. The falling film heat exchanger according to any one of claims 1 to 5, wherein the liquid dividers of the second heat exchanging section comprise a second cylindrical section connected to its cylindrical body.

7. The falling film heat exchanger according to claim 6, wherein the external diameter of the second cylindrical section of the liquid dividers of the second heat exchanging section is from 0.01 to 0.5 mm smaller than the internal diameter of the vertical tubes of the second heat exchanging section.

8. The falling film heat exchanger according to claim 6, wherein the internal diameter of the second cylindrical section of the liquid dividers of the second heat exchanging section is from 0.01 to 3.0 mm, or from 0.01 to 0.5 mm greater to the external diameter of the vertical tubes of the second heat exchanging section.

9. A method for heating up or cooling down a liquid composition, comprising the steps of:
a) providing a falling film heat exchanger according to any one of claims 1 to 8;
b) providing a heating fluid or a cooling fluid to the inlets of the bottom compartments of the first and second heat exchanging sections of the falling film heat exchanger;
c) providing the liquid composition to the inlet of the distribution section of the falling film heat exchanger and passing the liquid composition through the plurality of vertical tubes of the first and second heat exchanging sections;
d) collecting the heated or cooled liquid composition from the collection section of the falling film heat exchanger.

10. A method for removing ammonium carbamate from an aqueous composition, comprising the steps of:
a) providing a falling film heat exchanger according to any one of claims 1 to 8;
b) providing a heating fluid to the inlets of the bottom compartments of the first and second heat exchanging sections of the falling film heat exchanger;
c) providing the aqueous composition comprising ammonium carbamate to the inlet of the distribution section of the falling film heat exchanger and passing the aqueous composition comprising ammonium carbamate through the plurality of vertical tubes of the first and second heat exchanging sections;
d) collecting an aqueous composition depleted of ammonium carbamate from the collection section of the falling film heat exchanger.

11. The method according to claim 10, wherein the aqueous composition comprises urea.

12. Use of the falling film exchanger according to any one of claims 1 to 8 for removing ammonium carbamate from an aqueous solution comprising ammonium carbamate, and optionally urea.

13. Use of the falling film exchanger according to any one of claims 1 to 8 for removing water from an aqueous solution, particularly from an aqueous solution comprising urea.

## Patentansprüche

1. Fallfilmwärmetauscher, der einen Verteilungsabschnitt, einen ersten Wärmeaustauschabschnitt, der einen ersten Satz vertikaler Rohre umfasst, einen zweiten Wärmeaustauschabschnitt und einen Auffangabschnitt umfasst, wobei der erste und der zweite Wärmeaustauschabschnitt Folgendes umfassen:
- einen zylindrischen Körper, der eine obere Kammer und eine untere Kammer umfasst, die durch eine erste Scheibe, die mehrere Öffnungen umfasst, voneinander getrennt sind, wobei die obere Kammer ein offenes oberes Ende und die erste Scheibe an ihrem unteren Ende aufweist, und wobei die untere Kammer die erste Scheibe als ihr oberes Ende und eine untere Platte, die mehrere Öffnungen umfasst, als ihr unteres Ende aufweist, wobei die obere Kammer des zylindrischen Körpers dazu ausgelegt ist, eine Flüssigkeitszusammensetzung zu fassen, und die untere Kammer dazu ausgelegt ist, ein Heiz- oder Kühlfluid aufzunehmen, wobei die untere Kammer einen Fluideinlass für ein Heiz- oder Kühlfluid und einen Fluidauslass für ein Heiz- oder Kühlfluid umfasst; und
- mehrere vertikale Rohre, die innerhalb des zylindrischen Körpers angeordnet sind, wobei die Rohre zylindrisch mit einem konstanten Durchmesser und parallel zueinander sind, wobei jedes vertikale Rohr durch eine Öffnung der ersten Scheibe und durch eine Öffnung der unteren Platte aufgenommen ist und wobei jede Öffnung der ersten Scheibe und jede Öffnung der unteren Platte ein vertikales Rohr enthält, so dass die obere Kammer und die untere Kammer hermetisch voneinander getrennt sind;
wobei:
- der zweite Wärmeaustauschabschnitt mehrere Flüssigkeitsteiler umfasst, wobei ein Flüssigkeitsteiler auf jedem vertikalen Rohr der mehreren vertikalen Rohre des zweiten Wärmeaustauschabschnitts angeordnet ist;
- jeder Flüssigkeitsteiler des zweiten Wärmeaustauschabschnitts ein offenes oberes Ende aufweist;
- jeder Flüssigkeitsteiler des zweiten Wärmeaustauschabschnitts einen zylindrischen Körper umfasst, wobei der zylindrische Körper jedes Flüssigkeitsteilers eine oder mehrere Öffnungen umfasst;
- der erste Wärmeaustauschabschnitt und der zweite Wärmeaustauschabschnitt die gleiche Anzahl von vertikalen Rohren umfassen; und
- jedes vertikale Rohr des zweiten Wärmeaustauschabschnitts direkt unter einem vertikalen Rohr des ersten Wärmeaustauschabschnitts angeordnet ist.

2. Fallfilmwärmetauscher nach Anspruch 1, wobei jeder Flüssigkeitsteiler des zweiten Wärmeaustauschabschnitts vier Öffnungen in seinem zylindrischen Körper umfasst, insbesondere wobei die vier Öffnungen im gleichen Querschnitt des Flüssigkeitsteilers enthalten sind, insbesondere wobei die vier Öffnungen im gleichen Querschnitt des Flüssigkeitsteilers enthalten und um 90° beabstandet sind, wodurch ein Kreuz gebildet wird.

3. Fallfilmwärmetauscher nach Anspruch 1 oder 2, wobei der zweite Wärmeaustauschabschnitt ferner eine zweite Scheibe umfasst, die zwischen dem offenen oberen Ende des zylindrischen Körpers und der ersten Scheibe des zweiten Wärmeaustauschabschnitts angeordnet ist, wobei die zweite Scheibe mehrere erste Öffnungen zum Aufnehmen der mehreren Flüssigkeitsteiler des zweiten Wärmeaustauschabschnitts und mehrere zweite Öffnungen zum Ermöglichen eines Durchgangs der Flüssigkeitszusammensetzung durch die zweite Scheibe umfasst.

4. Fallfilmwärmetauscher nach einem der Ansprüche 1 bis 3, wobei der Innendurchmesser des oberen Endes der Flüssigkeitsteiler des zweiten Wärmeaustauschabschnitts 20 bis 80 % kleiner als der Innendurchmesser des zylindrischen Körpers der Flüssigkeitsteiler des zweiten Wärmeaustauschabschnitts ist.

5. Fallfilmwärmetauscher nach einem der Ansprüche 1 bis 4, wobei die eine oder die mehreren Öffnungen der Flüssigkeitsteiler des zweiten Wärmeaustauschabschnitts zwischen 30 und 100 mm über der ersten Scheibe des zweiten Wärmeaustauschabschnitts angeordnet sind.

6. Fallfilmwärmetauscher nach einem der Ansprüche 1 bis 5, wobei die Flüssigkeitsteiler des zweiten Wärmeaustauschabschnitts einen zweiten zylindrischen Abschnitt umfassen, der mit seinem zylindrischen Körper verbunden ist.

7. Fallfilmwärmetauscher nach Anspruch 6, wobei der Außendurchmesser des zweiten zylindrischen Abschnitts der Flüssigkeitsteiler des zweiten Wärmeaustauschabschnitts 0,01 bis 0,5 mm kleiner als der Innendurchmesser der vertikalen Rohre des zweiten Wärmeaustauschabschnitts ist.

8. Fallfilmwärmetauscher nach Anspruch 6, wobei der Innendurchmesser des zweiten zylindrischen Abschnitts der Flüssigkeitsteiler des zweiten Wärmeaustauschabschnitts 0,01 bis 3,0 mm oder 0,01 bis 0,5 mm größer als der Außendurchmesser der vertikalen Rohre des zweiten Wärmeaustauschabschnitts ist.

9. Verfahren zum Erwärmen oder Kühlen einer Flüssigkeitszusammensetzung, umfassend die folgenden Schritte:
a) Bereitstellen eines Fallfilmwärmetauschers nach einem der Ansprüche 1 bis 8;
b) Bereitstellen eines Heizfluids oder eines Kühlfluids zu den Einlässen der unteren Kammern des ersten und zweiten Wärmeaustauschabschnitts des Fallfilmwärmetauschers;
c) Bereitstellen der Flüssigkeitszusammensetzung zum Einlass des Verteilungsabschnitts des Fallfilmwärmetauschers und Leiten der Flüssigkeitszusammensetzung durch die mehreren vertikalen Rohre des ersten und des zweiten Wärmeaustauschabschnitts;
d) Auffangen der erwärmten oder gekühlten Flüssigkeitszusammensetzung aus dem Auffangabschnitt des Fallfilmwärmetauschers.

10. Verfahren zum Entfernen von Ammoniumcarbamat aus einer wässrigen Zusammensetzung, umfassend die folgenden Schritte:
a) Bereitstellen eines Fallfilmwärmetauschers nach einem der Ansprüche 1 bis 8;
b) Bereitstellen eines Heizfluids zu den Einlässen der unteren Kammern des ersten und des zweiten Wärmeaustauschabschnitts des Fallfilmwärmetauschers;
c) Bereitstellen der wässrigen Zusammensetzung, die Ammoniumcarbamat umfasst, zum Einlass des Verteilungsabschnitts des Fallfilmwärmetauschers und Leiten der wässrigen Zusammensetzung, die Ammoniumcarbamat umfasst, durch die mehreren vertikalen Rohre des ersten und des zweiten Wärmeaustauschabschnitts;
d) Auffangen einer wässrigen Zusammensetzung, der Ammoniumcarbamat entzogen wurde, aus dem Auffangabschnitt des Fallfilmwärmetauschers.

11. Verfahren nach Anspruch 10, wobei die wässrige Zusammensetzung Harnstoff umfasst.

12. Verwendung des Fallfilmtauschers nach einem der Ansprüche 1 bis 8 zum Entfernen von Ammoniumcarbamat aus einer wässrigen Lösung, die Ammoniumcarbamat und optional Harnstoff umfasst.

13. Verwendung des Fallfilmtauschers nach einem der Ansprüche 1 bis 8 zum Entfernen von Wasser aus einer wässrigen Lösung, insbesondere aus einer wässrigen Lösung, die Harnstoff umfasst.

## Revendications

1. Échangeur de chaleur à film de liquide tombant comprenant une section de distribution, une première section d'échange de chaleur comprenant un premier ensemble de tubes verticaux, une seconde section d'échange de chaleur, et une section de collecte, dans lequel les première et seconde sections d'échange de chaleur comprennent :
- un corps cylindrique, comprenant un compartiment supérieur et un compartiment inférieur séparés l'un de l'autre par un premier disque comprenant une pluralité d'ouvertures, dans lequel le compartiment supérieur a une extrémité supérieure ouverte et le premier disque à son extrémité inférieure, et dans lequel le compartiment inférieur a le premier disque en tant qu'extrémité supérieure et une plaque inférieure comprenant une pluralité d'ouvertures en tant qu'extrémité inférieure, dans lequel le compartiment supérieur du corps cylindrique est configuré pour contenir une composition liquide, et le compartiment inférieur est configuré pour recevoir un fluide de chauffage ou de refroidissement, dans lequel le compartiment inférieur comprend une entrée de fluide pour un fluide de chauffage ou de refroidissement, et une sortie de fluide pour un fluide de chauffage ou de refroidissement ; et
- une pluralité de tubes verticaux situés à l'intérieur du corps cylindrique, les tubes étant cylindriques avec un diamètre constant et parallèles les uns aux autres, dans lequel chaque tube vertical est reçu à travers une ouverture du premier disque et à travers une ouverture de la plaque inférieure et dans lequel chaque ouverture du premier disque et chaque ouverture de la plaque inférieure contient un tube vertical, de telle sorte que le compartiment supérieur et le compartiment inférieur sont hermétiquement séparés l'un de l'autre ;
dans lequel :
- la seconde section d'échange de chaleur comprend une pluralité de diviseurs de liquide, dans lequel un diviseur de liquide est situé par-dessus chaque tube vertical de la pluralité de tubes verticaux de la seconde section d'échange de chaleur ;
- chaque diviseur de liquide de la seconde section d'échange de chaleur a une extrémité supérieure ouverte ;
- chaque diviseur de liquide de la seconde section d'échange de chaleur comprend un corps cylindrique, dans lequel le corps cylindrique de chaque diviseur de liquide comprend une ou plusieurs ouvertures ;
- la première section d'échange de chaleur et la seconde section d'échange de chaleur comprennent le même nombre de tubes verticaux ; et
- chaque tube vertical de la seconde section d'échange de chaleur est situé directement en dessous d'un tube vertical de la première section d'échange de chaleur.

2. Échangeur de chaleur à film de liquide tombant selon la revendication 1, dans lequel chaque diviseur de liquide de la seconde section d'échange de chaleur comprend quatre ouvertures dans son corps cylindrique, en particulier dans lequel les quatre ouvertures sont comprises dans la même section transversale du diviseur de liquide, en particulier dans lequel les quatre ouvertures sont comprises dans la même section transversale du diviseur de liquide et sont espacées de 90°, formant ainsi une croix.

3. Échangeur de chaleur à film de liquide tombant selon la revendication 1 ou 2, dans lequel la seconde section d'échange de chaleur comprend en outre un second disque situé entre l'extrémité supérieure ouverte du corps cylindrique et le premier disque de la seconde section d'échange de chaleur, le second disque comprenant une pluralité de premières ouvertures pour recevoir la pluralité de diviseurs de liquide de la seconde section d'échange de chaleur, et une pluralité de secondes ouvertures pour permettre à la composition liquide de passer à travers le second disque.

4. Échangeur de chaleur à film de liquide tombant selon l'une quelconque des revendications 1 à 3, dans lequel le diamètre intérieur de l'extrémité supérieure des diviseurs de liquide de la seconde section d'échange de chaleur est de 20 à 80 % plus petit que le diamètre intérieur du corps cylindrique des diviseurs de liquide de la seconde section d'échange de chaleur.

5. Échangeur de chaleur à film de liquide tombant selon l'une quelconque des revendications 1 à 4, dans lequel l'une ou les plusieurs ouvertures des diviseurs de liquide de la seconde section d'échange de chaleur sont situées de 30 à 100 mm au-dessus du premier disque de la seconde section d'échange de chaleur.

6. Échangeur de chaleur à film de liquide tombant selon l'une quelconque des revendications 1 à 5, dans lequel les diviseurs de liquide de la seconde section d'échange de chaleur comprennent une seconde section cylindrique reliée à son corps cylindrique.

7. Échangeur de chaleur à film de liquide tombant selon la revendication 6, dans lequel le diamètre externe de la seconde section cylindrique des diviseurs de liquide de la seconde section d'échange de chaleur est de 0,01 à 0,5 mm plus petit que le diamètre interne des tubes verticaux de la seconde section d'échange de chaleur.

8. Échangeur de chaleur à film de liquide tombant selon la revendication 6, dans lequel le diamètre interne de la seconde section cylindrique des diviseurs de liquide de la seconde section d'échange de chaleur est de 0,01 à 3,0 mm, ou de 0,01 à 0,5 mm plus grand que le diamètre externe des tubes verticaux de la seconde section d'échange de chaleur.

9. Procédé pour chauffer ou refroidir une composition liquide, comprenant les étapes suivantes :
a) la fourniture d'un échangeur de chaleur à film de liquide tombant selon l'une quelconque des revendications 1 à 8 ;
b) la fourniture d'un fluide de chauffage ou d'un fluide de refroidissement aux entrées des compartiment inférieurs des première et seconde sections d'échange de chaleur de l'échangeur de chaleur à film de liquide tombant ;
c) la fourniture de la composition liquide à l'entrée de la section de distribution de l'échangeur de chaleur à film de liquide tombant et le passage de la composition liquide à travers la pluralité de tubes verticaux des première et seconde sections d'échange de chaleur ;
d) la collecte de la composition liquide chauffée ou refroidie, depuis la section de collecte de l'échangeur de chaleur à film de liquide tombant.

10. Procédé pour extraire du carbamate d'ammonium d'une composition aqueuse, comprenant les étapes suivantes :
a) la fourniture d'un échangeur de chaleur à film de liquide tombant selon l'une quelconque des revendications 1 à 8 ;
b) la fourniture d'un fluide de chauffage aux entrées des compartiment inférieurs des première et seconde sections d'échange de chaleur de l'échangeur de chaleur à film de liquide tombant ;
c) la fourniture de la composition aqueuse comprenant du carbamate d'ammonium à l'entrée de la section de distribution de l'échangeur de chaleur à film de liquide tombant et le passage de la composition aqueuse comprenant carbamate d'ammonium à travers la pluralité de tubes verticaux des première et seconde sections d'échange de chaleur ;
d) la collecte d'une composition aqueuse dont le carbamate d'ammonium a été extrait, depuis la section de collecte de l'échangeur de chaleur à film de liquide tombant.

11. Procédé selon la revendication 10, dans lequel la composition aqueuse comprend de l'urée.

12. Utilisation de l'échangeur à film de liquide tombant selon l'une quelconque des revendications 1 à 8 pour extraire du carbamate d'ammonium d'une solution aqueuse comprenant du carbamate d'ammonium, et facultativement de l'urée.

13. Utilisation de l'échangeur à film de liquide tombant selon l'une quelconque des revendications 1 à 8 pour extraire de l'eau d'une solution aqueuse, particulièrement d'une solution aqueuse comprenant de l'urée.
